# EUROPEAN PATENT SPECIFICATION

(11) **EP 3 213 708 B1**
(45) Date of publication and mention of the grant of the patent: **27.02.2019**
(21) Application number: 17151039.9
(22) Date of filing: 11.01.2017
(51) Int. Cl.: A61B 18/08, A61B 18/10

(54) **MEDICAL DEVICE**
MEDIZINISCHE VORRICHTUNG
DISPOSITIF MÉDICAL

(30) Priority: 15.01.2016 US 201662279098 P
(43) Date of publication of application: 06.09.2017
(73) Proprietor: Cook Medical Technologies LLC, Bloomington, IN 47404 (US)
(72) Inventor: ELGAARD, Per, 4690 Haslev (DK); PAAMAND, Rune Tore, 2700 Broenshoej (DK)
(74) Representative: Mathys & Squire LLP

(56) References cited:
- EP-A1- 1 706 052
- EP-A1- 2 853 216
- WO-A2-2008/002606
- US-A1- 2013 184 705

## Description

### FIELD

The present disclosure relates generally to medical devices. More specifically, the disclosure relates to a device for occluding or closing a body vessel using a current to heat and/or ablate the body vessel.

### BACKGROUND

There are numerous medical conditions when it is desired or necessary to close a body vessel, including the treatment of aneurysms, arteriovenous malformations, arteriovenous fistulas, for starving organs of oxygen and nutrients, in the treatment or containment of cancerous growths, and so on.

Several techniques are known and in use for closing or occluding such body vessels. Traditionally, vessels have been closed by means of external ligation, which generally must be carried out by an open surgery procedure, with its associated risks, inconvenience, and long patient recovery times. Other, more recent, methods aim to use an endoluminal procedure to insert into the vessel or organ one or more occlusion devices, such as a metal framed occluder, coils, pellets or the like, able to obstruct the flow of blood in the vessel.

It is also known to seek to constrict a vessel by endoluminal ablation, causing contraction of the vessel and/or coagulation of blood to form a blood clot in the vessel. Various methods can be employed to cause such ablation.

Reference is directed to EP 2 853 216 and EP 1 706 052 which disclose intravascular resistive heating elements with temperature sensing properties.

### BRIEF SUMMARY

The invention is defined in the appended claims. This disclosure provides a medical device and methods for conducting vessel ablation and occlusion.

In one approach, a method for providing resistive heating to a body vessel comprises the steps of: providing an elongate medical device having proximal and distal ends to a body vessel of a patient, wherein the medical device includes a resistive heating element disposed on the distal end of the heating device; wherein the resistive heating element comprises a metal material having thermistor properties, such that a resistance value of the resistive heating element will change as a function of a temperature of the resistive heating element; delivering a first power from a power source, the first power including a current, to the resistive heating element; determining the temperature of the resistive heating element as a function of the applied power; controlling the power applied to the resistive heating element to maintain the temperature of the heating element above a target temperature; determining a second power to apply to the resistive heating element to maintain the temperature of the heating element above the target temperature; detecting a power difference between the second power and the first power; determining that the detected power difference exceeds a threshold level; ceasing delivering of power in response to determining that the power difference exceeds a threshold level.

Various additional features and embodiments will become apparent with the following description. The present disclosure may be better understood by referencing the accompanying figures.

### BRIEF DESCRIPTION OF THE DRAWINGS

Fig. 1 depicts a partial, environmental view of a medical device in accordance with one aspect of the present disclosure;
Fig. 2A depicts a cross-sectional, side view of the device of Fig. 1;
Fig. 2B depicts a partial, blown-up, side view of the device of Fig. 1;
Fig. 3 depicts a side view of the device of Fig. 1;
Fig. 4 depicts a side view of the device of Fig. 1 delivered to a body cavity; and
Fig. 5 depicts steps of a method of use of the device of Fig. 1 in accordance with one aspect of the present disclosure.

### DETAILED DESCRIPTION

The present disclosure will now be described more fully with reference to the accompanying figures, which show various embodiments. The accompanying figures
are provided for general understanding of various embodiments and method steps. However, this disclosure may be embodied in many different forms. These figures should not be construed as limiting, and they are not necessarily to scale.

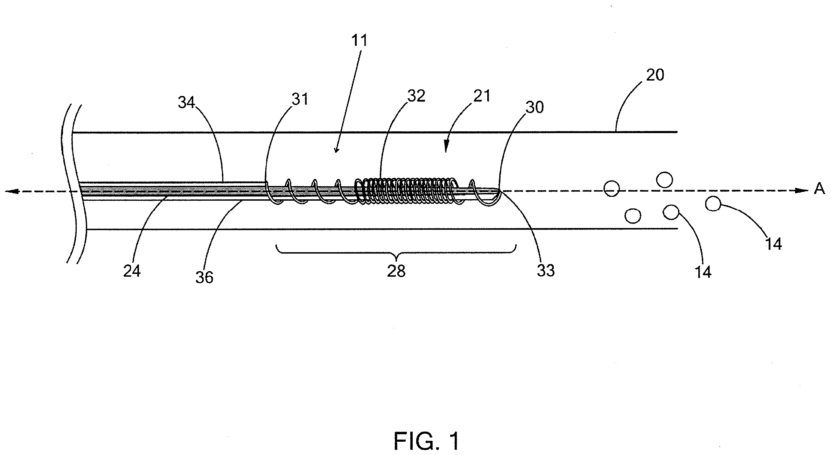

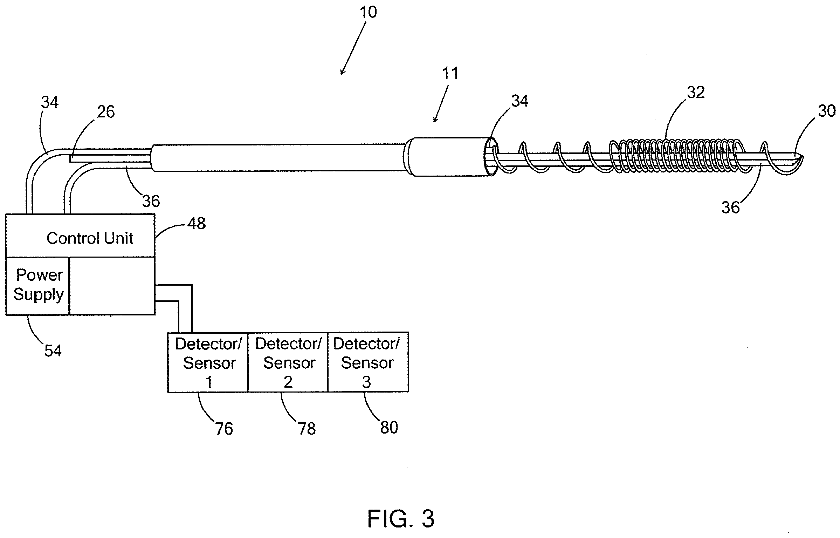

Fig. 1 depicts an environmental view of one embodiment of a medical device 11 for use in a system 10 that may be used to heat a body vessel at or adjacent a treatment site 21. The body vessel has a vessel wall 20. In this view, the device may be placed or positioned in the body vessel in a body. The device may have a support 24, or mandrel, that extends from a proximal end 26 (depicted in Fig. 3) to a distal end 30. The support 24 may define a longitudinal axis A. It will be appreciated that the illustrated environment of the device 11 and body vessel can differ. For example, the body vessel is illustrated as being generally tubular, but the device can be used to provide ablation or occlude other shapes of body vessels or body cavities. The device may have a curvature or be capable of being curved or bent similar to a common guidewire.

A resistive heating element, preferably in the form of a coil 32, may be disposed about the support 24, and the coil 32 may be electrically conductive. The electrical conductivity of the coil 32 allows for a current to be passed through the coil 32, resulting in the coil 32 becoming heated. The coil 32 may have a first end 31 being disposed between the proximal and distal ends (26, 30), and the coil 32 may extend to a second end 33 being disposed at the distal end 30. The support 24 may have a distal segment 28 that supports the coil 32, or about which the coil 32 is disposed.

The coil 32 may have a first coil spacing and a second coil spacing. The second coil spacing is such that the space between the coils of the coil 32 is smaller than the thickness of the coil 32 (meaning the coils are relatively close together, or touching). The first coil spacing is such that the space between the coils is greater than the thickness of the coils (meaning the coils are generally spaced apart from each other and not touching). This described spacing applies to particular embodiments, but other spacing or lack of spacing between adjacent coils could also be used. The spacing may be consistent or may vary along the length of the device.

The distal segment 28 may have an outer diameter that distally decreases to form a distal taper (as shown in Fig. 1). Alternatively, the support 24, including its distal segment 28, may have an outer diameter being uniform from the proximal end 26 to the distal end 30. The distal taper or tapered tip may provide the advantage of making the distal end 30 easier to track within the body vessel. Such distal taper may provide flexibility to track the device.

The device 11 may further have a first wire 34 and a second wire 36 for transferring the current through the coil 32. The first wire 34 may be electrically coupled or connected to a control unit 48 (depicted in Fig. 3). The first wire 34 may extend from the control unit 48 and along the longitudinal axis A to the first end 31 of the coil 32. The first wire 34 may be attached to the first end 31 of the coil 32 such that the connection provides an electrical and conductive coupling between the first wire 34 and the coil 32.

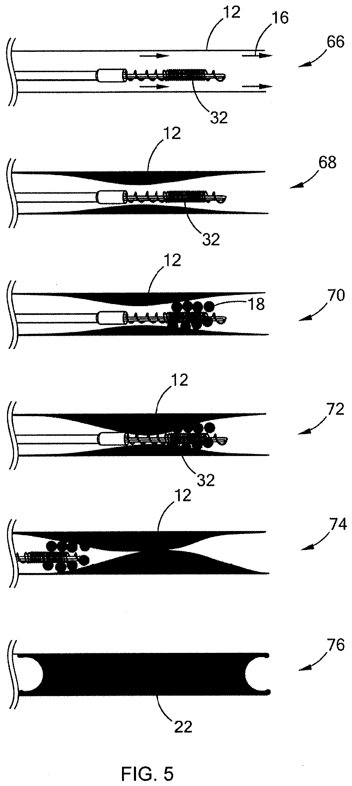

The second wire 36 may also be electrically coupled or connected to the control unit 48 and extend from the control unit 48, along the longitudinal axis A, to the second end 33 of the coil 32. The second wire 36 may be attached to the second end 33 such that the connection provides an electrical coupling between the second wire 36 and the coil 32. In this way, the device may form a first circuit along the path created by the control unit 48, the first wire 34, the coil 32, and the second wire 36. The device may form various circuits, which will be discussed further in Figs. 3-5. In Fig. 1, coagulated blood 14 may start to form as the device 11 operates, which will also be discussed further in Fig. 5.

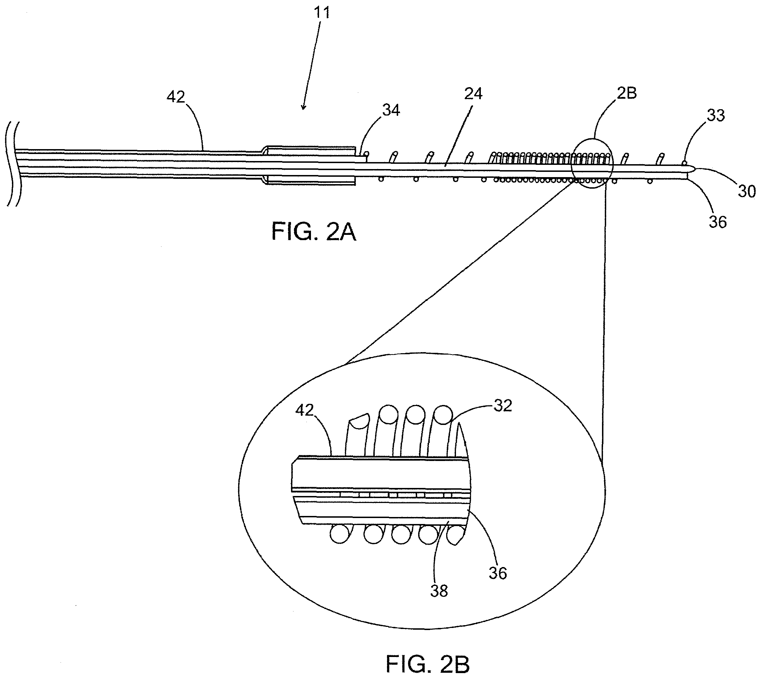

Figs. 2A-B depicts further details of the device. For example, Fig. 2A shows a cross-sectional view of the device. Fig. 2B shows a blown-up view of the device around circle 2B. In Fig. 2A, the support 24 has a uniform outer diameter from the proximal end 26 to the distal end 30. Additionally, either or both of the first and second wires (34, 36) may have an insulator 38 disposed about their outer surface to electrically insulate them from the rest of the device 11. Insulator 38 is shown disposed about the second wire 36 in Fig. 2B.

Additionally, the device 11 may have a shrink tubing disposed about the device 11. For example in Fig. 2A, the shrink tubing 42 may extend around the support 24, the first wire 34, the second wire 36, and/or any other portion of the device 11. As one advantage, the shrink tubing 42 may immobilize or bind the support 24, the first wire 34, and the second wire 36 in place so that they are immobilized relative to each other. The shrink tubing 42 may also immobilize and/or extend over a part of the coil 32 to keep the coil 32 in position as the device 11 is in use. Alternatively or additionally in Fig. 2B, the shrink tubing 42 may only be disposed about the support 24. In this configuration, the shrink tubing 42 may act to isolate or insulate the support 24 from the rest of the device 11.

Fig. 3 further depicts the proximal end 26 of the device and system 10. The proximal end 26 may be connected to the control unit 48 of the system 10, which is operatively coupled or connected to the first end of the coil 32 by way of the first wire 34. Additionally, the control unit 48 may be operatively coupled to the second end 33 of the coil 32 by way of the second wire 36. The control unit 48 may be operatively coupled to or include a power supply 54 to generate a voltage and a current. The power supply 54 could be a battery and/or other power supply capable of providing direct current and/or alternating current.

The power supply 54 may be operable by the control unit 48 and the user to transmit a current through the coil 32 when the device is in a resistive mode. The power supply 54 may further be operable to adjust the amount of voltage or current that is applied to the device 11 and the coil 32 in response to detecting various states of the device 11. The amount of current and/or voltage being applied to the device 11 by the power supply 54 can be moderated, controlled, altered, or the like by the control unit according to a predefined control algorithm or, alternatively, manually by a user. The operation of the control unit 48 and the power supply 54 in relation to the device 11 during its use will be described in further detail below.

The control unit 48 may also include an electrode drive unit (not shown) for moving the coil within the patient's vessel. This may also be done manually. In some embodiments, the control unit 48 may have or be coupled to a plurality of sensors and/or detectors (76, 78, 80) to determine different conditions of the device. For example, the plurality of sensors may be sensors selected from the group consisting of temperature sensors, current sensors, timers, impedance/resistance sensors, and pressure sensors. These various sensors may be used to detect and determine temperature, current, time, impedance/resistance, and/or pressure, respectively, to assist the user in using the device as the active components may not be visually accessible to the user. In another approach, the system 10 may be operated without the use of the sensors 76, 78, and 80 and may use the control unit 48 and power supply 54 to determine the state of the system 10 and device 11 during its use. In some approaches, the system 10 may not include these sensors and/or detectors, and the control unit 48 can determine various operating parameters of the system based on the detected functioning of the device 11 itself.

The control unit 48 may optionally include a user interface coupled to the control unit 48 and operable to provide data to the user and for input of user commands to the control unit 48. The user interface may, in its simplest embodiment, include an on/off switch for operating the control unit 48, and ablation and/or coagulation, with the control unit 48 then effecting the desired ablation process under the command of the control unit 48. In other embodiments, the user interface may be more sophisticated and enable, for example, a user to select different modes of ablation and optionally to produce, for instance, occluding barriers of different lengths, or occluding barriers or different sizes to accommodate different vessel sizing where ablation is desired.

The user interface also may have an output for providing ablation feedback and/or warning signals to a user. It may, for example, provide an indication of measured temperature and/or impedance, an indication of progress of ablation of the vessel and so on. For such purposes, the user interface may include a visual unit, for example a display to display quantitative data such as graphs, measures of temperature and impedance, determined length of occlusion and so on. In other embodiments, the display may be simpler, having for instance simple visual indicators such as one or more illuminated lamps. The output could also be an acoustic output and/or, as appropriate, a tactile output such as a vibration generator and so on. Any combination of user feedback devices may be provided.

When in use, the system 10 is preferably arranged to operate in a resistive heating mode for resistive heating and ablation. This mode may use the coil 32 to create a closed loop or circuit. The device is designed to create blood clotting, that is to heat the blood surrounding the electrical element, and/or to ablate the body vessel to close the vessel. This can be achieved by selecting an ablation energy level and/or an ablation time duration suitable to heat surrounding blood or tissue, which in some circumstances includes ablation of the vessel tunica, resulting in contraction of the vessel and occlusion as a result of the heating of the blood. The skilled person will be able to determine suitable ablation parameters from common general knowledge in the art.

It is to be appreciated that the level of power applied through the electrode or coil 32 and the time of application will be dependent upon factors including the size of the vessel, the amount and speed blood flow through the vessel, pulsation and turbulence of blood at the point of ablation, and so on.

In Fig. 3, elements of the system 10 may create various circuits. For example, Fig. 3 depicts elements that form a first circuit or electrical pathway for use in the resistive heating mode. Specifically, the first wire 34 may be electrically coupled to the control unit 48 and extend from the control unit 48 and along the longitudinal axis to the first end 31 of the coil 32. The first wire 34 may be attached to the first end 31 of the coil 32. The second wire 36 may also be electrically coupled to the control unit 48 and extend from the control unit 48 and along the longitudinal axis to the second end 33 of the coil 32. The second wire 36 may be attached to the second end 33 of the coil 32. The control unit 48, the first wire 34, the coil 32, and a second wire 36 form the first circuit being operable in the resistive mode.

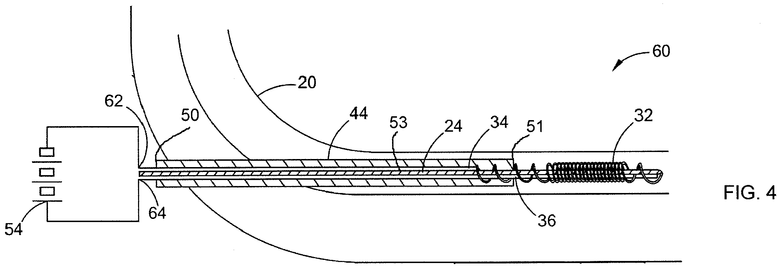

The system 10 may optionally include an outer sheath 44 for delivery and/or retrieval of the device 11 into and out of the body. The outer sheath 44 may optionally have a first sheath end 50 and extend to a second sheath end 51. The outer sheath 44 may form an inner lumen 53 therethrough from the first sheath end 50 to the second sheath end 51. As shown in Fig. 4, the support 24 may be slidably disposed or received within the inner lumen 53.

In one approach, the resistive part of the coil 32 is made from a material having thermistor properties. In this approach, the coil 32 is preferably made from a metal material with thermistor properties. One type of material having thermistor properties is Nifethal. A material with thermistor properties will have a resistance that will change as a function of temperature. For example, if the coil 32 becomes heated to a first temperature, it will have a first resistance corresponding to that temperature. If the coil 32 becomes heated to a second temperature, it will have a second resistance corresponding to the second temperature. The relationship between resistance and temperature can be linear or variable. In a linear relationship, temperature and resistance will vary according to a constant, where the constant can be positive or negative. For example, in a linear relationship, a positive constant is where the resistance of the coil increases as temperature increases. A negative constant is where the resistance of the coil decreases as temperature increases. A resistive coil without thermistor properties would have a constant of approximately zero, such that resistance will remain generally the same even as the material is heated. However, most metals will have some small degree of thermal dependence such that the constant is not zero. In a non-linear relationship, the concept of the positive/negative constant still applies to the relationship between temperature increase/decrease and resistance increase/decrease, but in a non-linear manner. Nifethal has a non-linear relationship. The precise relationship between temperature and resistance depends on the specific material properties of the selected material, which are known in the art depending on the material.

By using a coil 32 with thermistor properties, the current delivered to the coil 32 can be used as a measure of temperature of the coil 32. The temperature of the coil is dependent on multiple factors. One factor affecting the temperature of the coil 32 is the current passing through the coil 32. As is typical in resistive heating, as current passes through the coil 32, the coil 32 will become heated. Another factor of the temperature of the coil is the environment in which the coil 32 is disposed. In the case of the present system, the coil 32 is disposed within the body and within a body vessel where blood is flowing. More particularly, blood is being pumped through the body vessel.

As blood is pumped through the body vessel, the rate of the blood flow will operate to cool the coil 32 as the blood flows past the coil 32. In larger blood vessels, blood flow may be higher than in smaller blood vessels. For example, in an artery with a wider lumen, blood flow is less restricted than in an artery with a narrower lumen. Thus, the temperature of the coil 32 will additionally be affected by the rate of blood flow through the vessel.

During a resistive ablation procedure or occlusion of a blood vessel, the flow rate of the blood flowing through the vessel will decrease as the blood vessel becomes constricted. As the blood flow decreases, the cooling aspects of the flowing blood will also decrease, leading to a resultant increase in the temperature of the coil.

As stated above, the coil 32 has a resistance that is dependent on the temperature of the coil 32, so as the blood flow decreases and the coil temperature increases as a result, the resistance will also change. The control unit 48 can monitor and detect the resistance in the coil given the known voltage and current being applied, and can therefore monitor the temperature of the coil 32 based on these parameters without a separate temperature sensor.

Similarly, by detecting an increase in temperature of the coil, the controller can also determine that the blood flow has decreased, which is a result in the blood vessel becoming restricted. Accordingly, the control unit 48 can detect and determine that the occlusion is occurring.

The control unit 48 can be programmed manually or preprogrammed with the information necessary to determine the temperature of the coil 32, such as the particular thermistor material used for the coil 32, the size of the coil 32, the voltage and current being applied, and the like.

Power is delivered to the coil 32 as controlled by the control unit 48 and provided by the power supply 54. The power supply 54 can deliver the power via an adjustable DC transmission, where the amplitude of the DC transmission can be raised or lowered or held constant. In another approach, the power can be delivered as a pulse width modulated constant voltage. In this approach, a fixed voltage is delivered at varying periods of time, or in pulses. The duty cycle of the pulses can be adjusted to be more frequent, less frequent, at a longer duration, a shorter duration, or some combination of these variables to meter the amount of power that is delivered over a certain period of time. In some cases, controlling the power via pulse width modulation is simpler to make and more efficient, as it can be easier to control on/off rather than adjusting an amplitude of the voltage. While the disclosed voltage, current, and power are described as being in the form of a DC transmission, an AC transmission of voltage, current, and power could also be used. In one approach, the power supply 54 is an AC power source higher than 200 kHz to reduce nerve stimulation.

In one approach to resistive heating, it may be desirable to maintain the coil 32 at a constant or desired temperature, or to control the temperature of the coil 32 in the event the temperature of the coil deviates from the intended or desired temperature. It will be appreciated that a reference to maintaining a constant temperature is intended to encompass a constant temperature and adjusting to the constant temperature, even though temperature may deviate slightly from the desired temperature during the temperature control.

The maintenance of a desired temperature can be desirable in resistive heating for ablation and occlusion of a blood vessel. For instance, without temperature control, the coil 32 could become hotter than desired as the blood flow decreases when the body vessel becomes partially or fully occluded. A higher than desired temperature may result in patient discomfort or other undesirable effects, such as boiling of the blood and/or charring at the surface of the device.

The resistive part of the coil 32 is configured so that the application of power to the wires (34, 36) causes current to flow through the resistive part, which can cause heating of the resistive part. This, in turn, causes embolization and/or ablation of blood surrounding the resistive part, and/or heating and consequential contraction of the vessel in the vicinity of the resistive part.

Fig. 4 depicts the resistive heating mode or resistive mode 60. As described above in Fig. 3, a power supply 54 may generate a current to transmit through the coil 32. The current may be transmitted through the first circuit, including the power supply 54, the first wire 34, the coil 32, and the second wire 36. As the current flows through the coil 32, the coil 32 heats up. This may heat the vessel wall 20, causing coagulation and occlusion

The first wire 34 may be attached via a first lead 62 to a power supply 54 (e.g. a battery). Additionally, the second wire 36 may be attached to the power supply 54 through a second lead 64. Various conductive wires and connectors may be used to electrically couple parts of the device.

Fig. 5 depicts a method to fully occlude a body vessel 12. In step 66, the device may be positioned in the body vessel 12 adjacent the treatment site. At this time, blood flow 16 will be substantially normal and flowing at a first rate. In step 68, the system 10 may generate power from the control unit 48 and the power source 54, causing current to flow through the first circuit into the coil 32 and heat the coil 32.

When the current is flowing, the method may include heating a local zone of the body vessel 12 after the step of generating the power. This step of heating may cause swelling of the body vessel 12 at the treatment site, as depicted in step 68. As shown, the step 68 of first transmitting power may comprise avoiding contact of the coil 32 with the vessel wall 12. However, it is preferred in resistive heating that the coil 32 contact the vessel wall 20 to provide direct contact between the coil 32 and the wall to heat the wall 20 and cause it to swell.

In step 70, blood 18 may start to coagulate and occlude the body vessel 20. The blood may coagulate on the coil 32 in some instances.

In step 72, the device 11 will continue to generate the current with the power supply 54. As the vessel wall 12 swells a further amount, the step of transmitting a current through the coil may comprise contacting the vessel wall with the coil or contacting additional surface area of the vessel wall.

In step 74, the device 11 may start to fully occlude the body vessel 12. As such, the device may start to withdraw from the body vessel 12. This withdrawal may be manual or automatic. In step 76, the body vessel has been fully included with occlusion 22. The device may be fully withdrawn.

The above described steps 66-76 provide a general description of resistive heating with the coil 32. The use of the coil 32 having thermistor properties along with the control unit 48 and the power supply 54 can provide for further control of the device 11 to control the temperature of the coil 32 during an ablation or occlusion procedure, as further described below.

In one approach, a method for occluding the blood vessel 12 includes providing the device 11 to the body vessel, as described above in step 66. The device 11 includes the coil 32 having thermistor properties. Power is delivered to the device 11 and in particular the coil 32, with the power that is provided including a current.

In use, the device 11 will have a desired temperature or target temperature for the coil 32 to be heated to. Upon application of power to the device 11, the coil 32 can be heated until it reaches the desired temperature. In one example, the desired temperature can be approximately 90 degrees C. An initial steady state of the coil 32 can be reached, which is a function of the required power to overcome the heat sink effect of the flowing blood to maintain the desired temperature.

Thus, a first power is delivered from the power supply 54, with the first power including a current, to the coil 32 at a first time. With the first power and current being applied, the control unit determines the temperature of the coil 32 as a function of the applied power. The control unit 48 can determine the voltage and current that is being applied with the first power, and will therefore determine the resistance of the coil 32 during the application of the first power. By determining the resistance, the control unit 48 can determine the temperature of the coil 32 based on the particular thermistor properties of the coil. In some instances, the temperature of the coil 32 may be inhomogeneous. The various thresholds and calculations performed by the control unit can be configured to compensate for such inhomogeneous temperatures.

The control unit 48 will then control the power supply 54 to adjust the voltage and current accordingly if the temperature is higher than the desired temperature or lower than the desired temperature. For example, if the temperature is too low, more power will be applied, or if the temperature is too high, less power will be applied.

The amount of power to be applied can be adjusted by changing the amplitude of the DC or AC signal or the duty cycle of a constant voltage pulse width modulation. The power applied to the coil 32 can be controlled to maintain the temperature above the predetermined or target/desired temperature.

The control unit 48 continues to monitor the temperature based on the resistance detected as a function of voltage and current, and will then determine a second power to apply to the resistive heating element to maintain the temperature above the predetermined level.

As occlusion occurs, as shown in steps 68-72, blood flow will decrease, and the heat sink effects of the blood flow will reduce. Accordingly, less power is necessary to maintain the temperature of the coil 32 at or above the predetermined temperature. Thus, the control unit 48 can also detect a power difference between the first power and the second power. The first power is higher because the blood is flowing more freely prior to occlusion. The second power is lower because the blood is flowing more slowly due to the occlusion that is occurring.

The control unit 48 can continually monitor the power difference that is determined. During an initial stage of occlusion, the power difference can be relatively small because blood continues to flow. During a late stage of occlusion, as shown in step 74, or at the end of a complete or substantially complete occlusion, the power difference will be relatively high. The control unit 48 can determine if the power difference exceeds a threshold level, indicating a large drop in the required power to maintain the coil 32 at or above the predetermined temperature.

In response to determining the power difference exceeding a threshold level, the control unit 48 can command the power source 54 to stop transmitting power to the coil 32. This allows the system to automatically stop applying power and heating the coil 32 at the conclusion of occlusion without requiring other confirmation of occlusion, such as using blood flow monitors, visualization, or other methods of monitoring occlusion.

It should be understood that the foregoing relates to exemplary embodiments of the disclosure and that modifications may be made without departing from the scope of the following claims. While the disclosure has been described with respect to certain embodiments it will be appreciated that modifications and changes may be made by those skilled in the art. Whilst examples have been given of resistive heating provided to a body vessel, it should be understood that the invention applies to other examples of resistive heating at a location, to include non-surgical and non-therapeutic examples of heating in a rig or model which simulates a body vessel for the purposes of training, demonstrating or trialing systems or devices for resistive heating in a body vessel.

## Claims

1. A system for providing resistive heating to a body vessel, the system comprising:
an elongate medical device (11) having proximal and distal ends to a body vessel of a patient, wherein the medical device includes a resistive heating element (32) disposed on the distal end of the heating device;
wherein the resistive heating element comprises a metal material having thermistor properties, such that a resistance value of the resistive heating element will change as a function of a temperature of the resistive heating element; and
a controller (48) adapted for
delivering a first power from a power source, the first power including a current, to the resistive heating element;
determining the temperature of the resistive heating element as a function of the applied power;
controlling the power applied to the resistive heating element to maintain the temperature of the heating element above a target temperature;
determining a second power to apply to the resistive heating element to maintain the temperature of the heating element above the target temperature;
detecting a power difference between the second power and the first power;
determining that the detected power difference exceeds a threshold level;
ceasing delivering of power in response to determining that the power difference exceeds a threshold level.

2. The system of claim 1, wherein the second power is lower than the first power.

3. The system of any one of the preceding claims, wherein the power is delivered in the form of an adjustable AC or DC signal, preferably wherein the amplitude of the AC or DC signal is lowered to reduce the power delivered to the heating element.

4. The system of any one of the preceding claims, wherein the power is delivered in the form of a pulse width modulated constant voltage, preferably wherein the duty cycle of the constant voltage is reduced to reduce the power delivered to the heating element.

5. The system of any one of the preceding claims, wherein the heating element comprises a coil extending around an outer surface of an elongate shaft at a distal end thereof.

6. The system of any one of the preceding claims, the controller being further adapted for defining an initial steady state having a first voltage, a first resistance, and a first current corresponding to the first power.

7. The system of claim 6, the controller being further adapted for detecting the resistance in the heating element and preferably determining the temperature of the coil based on the detecting resistance; or detecting an increase in the resistance of the coil and determining an increase in the temperature corresponding to the increase in the resistance.

8. The system of any one of the preceding claims, the controller being further adapted for determining a decrease in blood flow at the heating element in response to determining the second power.

9. The system of any one of the preceding claims, wherein the power source is an AC power source higher than 200 kHz.

## Patentansprüche

1. System, um einem Körpergefäß Widerstandsheizung bereitzustellen, wobei das System umfasst:
eine längliche medizinische Vorrichtung (11) mit proximalen und distalen Enden an einem Körpergefäß eines Patienten, wobei die medizinische Vorrichtung ein Widerstandsheizelement (32) einschließt, das an dem distalen Ende der Heizvorrichtung angeordnet ist;
wobei das Widerstandsheizelement ein Metallmaterial mit Thermistoreigenschaften umfasst, so dass ein Widerstandswert des Widerstandsheizelements sich als Funktion einer Temperatur des Widerstandsheizelements ändert; und
eine Steuerung (48), die eingerichtet ist zum Zuführen einer ersten Leistung aus einer Leistungsquelle an das Widerstandsheizelement, wobei die erste Leistung einen Strom einschließt;
Ermitteln der Temperatur des Widerstandsheizelements als Funktion der angelegten Leistung;
Steuern der an das Widerstandsheizelement angelegten Leistung, um die Temperatur des Heizelements über einer Zieltemperatur zu halten;
Ermitteln einer zweiten Leistung, die an das Widerstandsheizelement angelegt werden soll, um die Temperatur des Heizelements über der Zieltemperatur zu halten;
Detektieren einer Leistungsdifferenz zwischen der zweiten Leistung und der ersten Leistung;
Ermitteln, ob die detektierte Leistungsdifferenz ein Schwellenwertniveau übersteigt;
Beenden des Zuführens der Leistung in Reaktion auf die Ermittlung, dass die Leistungsdifferenz ein Schwellenwertniveau übersteigt.

2. System nach Anspruch 1, wobei die zweite Leistung niedriger als die erste Leistung ist.

3. System nach einem der vorhergehenden Ansprüche, wobei die Leistung in Form eines anpassbaren AC- oder DC-Signals zugeführt wird, wobei vorzugsweise die Amplitude des AC- oder DC-Signals abgesenkt wird, um die dem Heizelement zugeführte Leistung zu reduzieren.

4. System nach einem der vorhergehenden Ansprüche, wobei die Leistung in Form einer pulsbreitenmodulierten konstanten Spannung zugeführt wird, wobei vorzugsweise das Tastverhältnis der Konstantspannung abgesenkt wird, um die dem Heizelement zugeführte Leistung zu reduzieren.

5. System nach einem der vorhergehenden Ansprüche, wobei das Heizelement eine Spule umfasst, die sich um eine Außenfläche eines länglichen Schafts an dessen distalem Ende erstreckt.

6. System nach einem der vorhergehenden Ansprüche, wobei die Steuerung ferner eingerichtet ist, um einen anfänglichen Gleichgewichtszustand mit einer ersten Spannung, einem ersten Widerstand und einem ersten Strom zu definieren, welche der ersten Leistung entsprechen.

7. System nach Anspruch 6, wobei die Steuerung ferner eingerichtet ist, um den Widerstand in dem Heizelement zu detektieren und vorzugsweise die Temperatur der Spule basierend auf dem Detektieren des Widerstands zu ermitteln; oder einen Anstieg des Widerstands der Spule zu detektieren und einen Anstieg der Temperatur entsprechen dem Anstieg des Widerstands zu ermitteln.

8. System nach einem der vorhergehenden Ansprüche, wobei die Steuerung ferner eingerichtet ist, um eine Abnahme des Blutflusses an dem Heizelement in Reaktion auf das Ermitteln der zweiten Leistung zu ermitteln.

9. System nach einem der vorhergehenden Ansprüche, wobei die Leistungsquelle eine AC-Leistungsquelle mit mehr als 200 kHz ist.

## Revendications

1. Système fournissant un chauffage résistif à un vaisseau corporel, le système comprenant :
un dispositif médical allongé (11) possédant des extrémités proximale et distale par rapport à un vaisseau corporel d'un patient, le dispositif médical comprenant un élément chauffant résistif (32) disposé sur l'extrémité distale du dispositif chauffant ;
dans lequel l'élément chauffant résistif comprend un matériau métallique possédant des propriétés de thermistance, de sorte qu'une valeur de résistance de l'élément chauffant résistif change en fonction d'une température de l'élément chauffant résistif ; et
un organe de commande (48) conçu pour
délivrer une première puissance provenant d'une source d'alimentation, la première puissance comprenant un courant, à l'élément chauffant résistif ;
déterminer la température de l'élément chauffant résistif en fonction de la puissance appliquée ;
commander la puissance appliquée à l'élément chauffant résistif pour maintenir la température de l'élément chauffant au-dessus d'une température cible ;
déterminer une seconde puissance à appliquer à l'élément chauffant résistif pour maintenir la température de l'élément chauffant au-dessus de la température cible ;
détecter une différence de puissance entre la seconde puissance et la première puissance ;
déterminer que la différence de puissance détectée dépasse un niveau seuil ;
arrêter d'administrer la puissance en réponse à la détermination que la différence de puissance dépasse un niveau seuil.

2. Système selon la revendication 1, dans lequel la seconde puissance est inférieure à la première puissance.

3. Système selon l'une quelconque des revendications précédentes, dans lequel la puissance est administrée sous forme d'un signal c.a. ou c.c. réglable, de préférence dans lequel l'amplitude du signal c.a. ou c.c. est abaissée pour réduire la puissance délivrée à l'élément chauffant.

4. Système selon l'une quelconque des revendications précédentes, dans lequel la puissance est administrée sous forme d'une tension constante à modulation d'impulsions en durée, de préférence dans lequel le cycle opératoire de la tension constante est diminué pour réduire la puissance délivrée à l'élément chauffant.

5. Système selon l'une quelconque des revendications précédentes, dans lequel l'élément chauffant comprend une bobine s'étendant autour d'une surface externe d'un arbre allongé au niveau d'une extrémité distale de celui-ci.

6. Système selon l'une quelconque des revendications précédentes, l'organe de commande étant en outre conçu pour définir un régime permanent initial possédant une première tension, une première résistance et un premier courant correspondant à la première puissance.

7. Système selon la revendication 6, l'organe de commande étant en outre conçu pour détecter la résistance dans l'élément chauffant et de préférence pour déterminer la température de la bobine sur la base de la détection de la résistance ; ou pour détecter une augmentation de la résistance de la bobine et pour déterminer une augmentation de la température correspondant à l'augmentation de la résistance.

8. Système selon l'une quelconque des revendications précédentes, l'organe de commande étant en outre conçu pour déterminer une diminution du flux sanguin au niveau de l'élément chauffant en réponse à la détermination de la seconde puissance.

9. Système selon l'une quelconque des revendications précédentes, dans lequel la source d'alimentation est une source de courant alternatif supérieure à 200 kHz.
